Europäisches Patentamt

European Patent Office

Office européen des brevets

(11) Veröffentlichungsnummer: **0 266 451**
B1

# (12) EUROPÄISCHE PATENTSCHRIFT

(45) Veröffentlichungstag der Patentschrift:
14.02.90

(21) Anmeldenummer: 86115357.5

(22) Anmeldetag: 05.11.86

(51) Int. Cl.⁴: **A61F 9/00**

(54) Okulopressionsgerät.

(43) Veröffentlichungstag der Anmeldung:
11.05.88 Patentblatt 88/19

(45) Bekanntmachung des Hinweises auf die Patenterteilung:
14.02.90 Patentblatt 90/7

(84) Benannte Vertragsstaaten:
BE CH DE FR GB IT LI SE

(56) Entgegenhaltungen:
EP-A- 0 180 317
DE-C- 1 055 175
FR-A- 2 395 742
US-A- 3 527 204

(73) Patentinhaber: BOSCH + SOHN GmbH u. Co.KG Fabrik medizinischer Apparate, Bahnhofstrasse 64,
D-7455 Jungingen(DE)

(72) Erfinder: Klein, Leonhard, Blumenstrasse 5,
D-6900 Heidelberg(DE)

(74) Vertreter: Patentanwälte TER MEER - MÜLLER - STEINMEISTER, Mauerkircherstrasse 45,
D-8000 München 80(DE)

## Beschreibung

Die Erfindung betrifft ein Okulopressionsgerät mit einer an eine Pumpe angeschlossenen und auf dem Auge eines Probanden zu befestigenden Pelotte, wie es beispielsweise aus FR-A 2 395 742 bekannt ist.

Die Okulopression dient zur Vorbereitung für die Ophthalmochirurgie, insbesondere bei der Implantation einer Intraokularlinse, wo ein hypotoner Bulbus mit tiefer Vorderkammer gefordert ist. Um den Flüssigkeitsdruck im Auge zu reduzieren, wird eine Pelotte mit einem Band auf dem Auge befestigt und sodann wird die Pelotte mittels eines Pumpballs unter Kontrolle durch ein Manometer aufgepumpt, so daß auf das Auge ein Druck zwischen 25 und 40 mmHg ausgeübt wird. Während der etwa 15 Minuten dauernden Okulopression wird die intraokulare Flüssigkeit allmählich aus dem Auge gedrückt und es entsteht ein sogenanntes "weiches Auge". Beim Nachgeben des Auges dehnt sich die Pelotte aus, was aufgrund des geringen Volumens der Pelotte zu einem Druckabfall führt, der unerwünscht und nachteilig ist. Während der Okulopression ist es also erforderlich, daß der Proband unter ständiger Kontrolle bleibt; der Druck muß wiederholt manuell nachgeregelt werden. Dabei ergeben sich in der Praxis insbesondere bei ungeübtem Personal erhebliche Probleme, da es äußerst schwierig ist, bei dem sehr kleinen Volumen der Pelotte derart niedrige Druckwerte zwischen 25 und 40 mmHg manuell exakt einzustellen. Da bereits bei einem Druck von etwa 55 mmHg eine Schädigung des Auges zu befürchten ist, bedarf die bisher übliche manuelle Handhabung des Pressionsgeräts einer sehr großen ärztlichen Erfahrung, um einerseits eine Schädigung des Auges zu vermeiden und um andererseits während der Okulopression einen ganz konstanten Druck auf dem Auge aufrechtzuerhalten. Wird ein durch das nachgebende Auge verursachter Druckverlust nicht unmittelbar durch Nachpumpen ausgeglichen, so hat dies ein schlechteres Ergebnis der Okulopression zur Folge, was für den Chirurgen eine unliebsame Reduzierung der ihm zur Verfügung stehenden Operationszeit bedeuten kann.

Der Erfindung liegt damit die Aufgabe zugrunde, ein Okulopressionsgerät zu schaffen, mit dem sich die Okulopression bei konstantem Druck über im Prinzip beliebig lange Zeiträume ohne Gefahr einer Schädigung des Auges durchführen läßt.

Ein erfindungsgemäßes Okulopressionsgerät der eingangs genannten Art weist die im kennzeichnenden Teil des Patentanspruchs 1 angegebenen Merkmale auf.

Bei einem aus der Druckschrift EP-A 0 180 317 bekannten chirurgischen Gerät zur operativen Entfernung von Wucherungen im Augeninneren ist zwar eine mikroprozessorgesteuerte Drucküberwachungseinrichtung vorgesehen, die über eine beispielsweise in die Augenvorkammer zu stechende Hohlnadel eine unter dem erwünschten Augeninnendruck gehaltene physiologische Kochsalzlösung in das Augeninnere drückt.

Die Salzlösung wird unter der Wirkung eine Peristaltikpumpe in einem Umlaufsystem durch Regelung der Drehzahl der Pumpe auf dem erwünschten konstanten Druck gehalten. Sinkt der über die Hohlnadel im Augeninneren erfaßte Druck ab, so wird die Pumpleistung erhöht, bei ansteigendem Augeninnendruck dagegen erniedrigt. Bei diesem bekannten Augenoperationsgerät handelt es sich jedoch um ein insgesamt aufwendiges System, bei dem der unmittelbar im Augeninneren gemessene Druck über die Einspeisung bzw. Entnahme von Augenflüssigkeit/Salzlösung indirekt über die Regulierung der Drehzahl eines Pumpensystems konstant gehalten bzw. auf gewünschte Werte geregelt wird. Abgesehen davon, daß in EP-A 0 180 317 nicht beschrieben ist, welche Regelkriterien für das umlaufende Medium (Kochsalzlösung) gelten und wie die Regeleinrichtung zu gestalten wäre, kommt für den hier vorgesehenen Zweck der Okulopression ein aufwendiges Regelsystem mit umlaufendem Medium kaum in Frage.

Wie bei bekannten Okulopressionsgeräten auch, wird bei der erfindungsgemäßen Verbesserung zunächst eine bekannte und übliche Pelotte mittels eines um den Kopf des Probanden geschlungenen Bandes auf dem Auge befestigt. Sodann wird schalterbetätigt die Pumpe mit angeschlossener Pelotte in Betrieb gesetzt, bis der Sollwert erreicht ist. Sinkt der Druck in der Pelotte bei sich langsam entleerendem Auge ab, wird also der Sollwert geringfügig unterschritten, so wird die Pumpe wieder eingeschaltet, bis das Drucksignal vom Druckwandler den Sollwert beispielsweise 30 mmHg, wieder erreicht. Der andere Komparator ist eingangsseitig einerseits mit dem Sollwert und andererseits mit einem um ein festes Verhältnis reduzierten Anteil (z. B. 0,9) oder einem festen, 3 bis 4 mmHg entsprechenden geringeren Betrag des Druckwandlersignals beaufschlagt und liefert ausgangsseitig ein Steuersignal an das Reduzierventil, sobald der Druck in der Pelotte den Sollwert beispielsweise bei gestörtem ersten Komparator um den Differenzwert zwischen Sollwert und dem reduzierten Drucksignal überschreitet.

Dies ist selbstverständlich auch der Fall, wenn der Druck in der Pelotte und damit der Druck auf das Auge beispielsweise auf 25 mmHg reduziert werden soll.

Die Erfindung und vorteilhafte Einzelheiten werden nachfolgend unter Bezug auf die Zeichnung anhand eines Ausführungsbeispiels näher erläutert. Es zeigen:

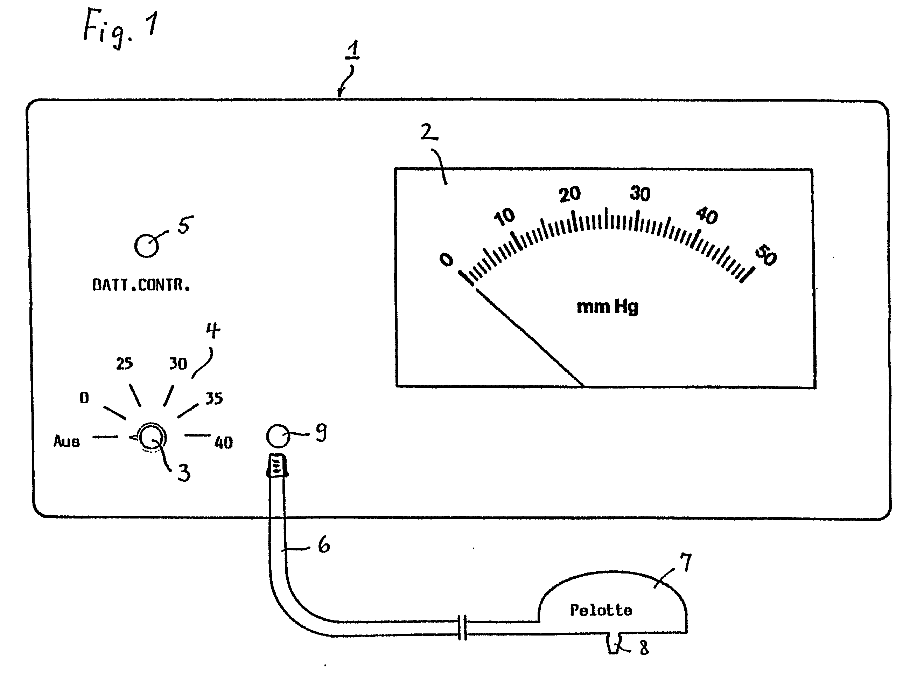

Fig. 1 die (schematische) Frontansicht eines Okulopressionsgeräts gemäß der Erfindung, und

Fig. 2 den prinzipiellen schaltungstechnischen Aufbau des Geräts nach Fig. 1.

Von der Außenseite wird ein Okulopressionsgerät 1 mit erfindungsgemäßen Merkmalen mit einem Druckanzeigeinstrument 2 versehen sein, auf dessen Skala die für die Okulopression üblichen Druckwerte anzeigbar sind. An einem Wahlschalter 3 einer zur Druckvorwahl bestimmten Schaltereinrichtung 20 können bestimmte Drucksollwerte 4 in üblicher Weise gewünschten Druckwertstufen zwischen beispielsweise 25 und 40 mmHg vorgewählt werden. Eine Lichtanzeige 5 dient zur Anzeige der Betriebsbereitschaft bzw. zur Kontrolle des Ladezustands einer Batterie, da es für die universelle Einsatzbereitschaft des Geräts zweckmäßig sein wird, sowohl Netzbetrieb als auch Batteriebetrieb vorzusehen. An einem Druckluftauslaß 9 kann über einen Verbindungsschlauch 6 eine übliche Pelotte 7 angeschlossen werden, die in bekannter Weise mittels eines (nicht gezeigten) Bandes über einen Ansatz 8 auf dem Auge befestigt werden kann.

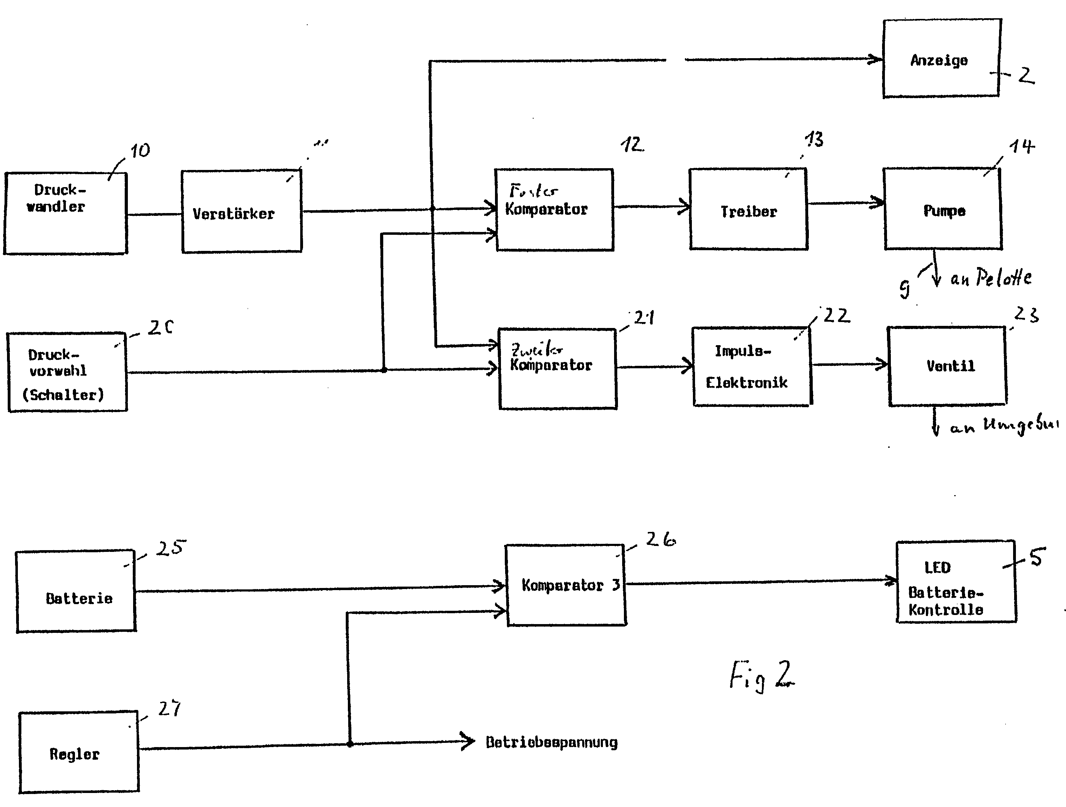

Die Blockschaltbilddarstellung der Fig. 2 läßt den prinzipiellen Aufbau der elektronischen Steuer- und Überwachungseinrichtung zur Einstellung und Konstanthaltung des Drucks in der Pelotte erkennen.

Ein Druckwandler 10 erfaßt den Druck im Schlauch 6 bzw. in der Pelotte 7, also auf dem Verbindungsweg zwischen einer elektrischen Pumpe 14 und der Pelotte 7. Das über einen Verstärker 11 verstärkte Druckwandlersignal beaufschlagt einerseits den Vergleichseingang eines ersten Komparators 12 und andererseits den Vergleichseingang eines zweiten Komparators 21. Zur unmittelbaren Anzeige gelangt das Druckwandlersignal außerdem auf die Druckanzeigeeinrichtung 2. Der Vergleichseingang des zweiten Komparators 21 wird jedoch nicht durch das Druckwandlersignal unmittelbar, sondern mit einem um ein fest eingestelltes Verhältnis reduzierten Druckwert beaufschlagt. Der Reduktionsfaktor bestimmt zu einem wesentlichen Teil die Empfindlichkeit der Einstellung des Druckwerts in der Pelotte 7, wie noch später erläutert werden wird. Für die Praxis zeigt es sich als empfehlenswert, aber auch als ausreichend, den Reduktionsfaktor auf 0.9 einzustellen, d.h. der Vergleichseingang des zweiten Komparators 21 wird mit einem um 10 % im Pegel reduzierten Druckwandlersignal beaufschlagt.

Der Soll- oder Einstellwert des Drucks wird an der Schaltereinrichtung 20 mittels des Wählknopfs 3 eingestellt, beispielsweise auf einen Druckwert von 35 mmHg. Das diesem Sollwert entsprechende elektrische Signal beaufschlagt die Solleingänge der Komparatoren 12 und 21.

Dem ersten Komparator 12 ist ein Treiber 13 für die Pumpe 14 nachgeschaltet, während der Komparator 21 das Ansteuersignal für eine Impulselektronikschaltung 22 liefert, die das Reduzierventil 23 erregt.

Der Vollständigkeit halber zeigt die Fig. 2 noch den Aufbau des Stromversorgungsteils des Gesamtgeräts. Wegen der erforderlichen Präzision der Drucküberwachung und der erwünschten sehr kleinen Schwankungsbreite des eingestellten Drucks in der Pelotte 7 wird ein geregeltes Versorgungsteil 27 erforderlich sein. Ein dritter Komparator 26 vergleicht eine von einer Batterie 25 gelieferte Spannung gegen die geregelte Spannung vom Regler 27 und schaltet bei Unterspannung die Batteriekontrollanzeige 5 aus, die beispielsweise mittels einer lichtemittierenden Diode verwirklicht sein kann.

Die Schaltung nach Fig. 2 arbeitet wie folgt:

Solange in der Pelotte 7 ein an der Druckvorwahl-Schaltereinrichtung 20 eingestellter Druckwert noch nicht erreicht ist, liefert der Komparator 12 über den Treiber 13 ein Erregersignal an die Pumpe 14, die damit den Druck in der Pelotte 7 erhöht. Sobald das vom Druckwandler 10 gelieferte Drucksignal den eingestellten Sollwert des Drucks erreicht, sperrt der erste Komparator 12 den Treiber 14, so daß die Pumpe 14 abgeschaltet wird. Wird das unter dem Überdruck der Pelotte 7 stehende Auge allmählich weich, so sinkt das Drucksignal ab und der erste Komparator 12 schaltet die Pumpe 14 wieder so lange ein, bis der an der Schaltereinrichtung 20 eingestellte Sollwert wieder erreicht ist. Auf diese Weise kann der Druck auf das Auge innerhalb eines Schwankungsbereichs von maximal ± 2 mmHg konstant gehalten werden.

Am Solleingang des zweiten Komparators 21 liegt einerseits das Druckvorwahlsignal von der Schaltereinrichtung 20, während den Vergleichseingang andererseits voraussetzungsgemäß der in einem festgelegten Verhältnis oder um einen festgelegten Betrag untersetzte Druckwert vom Druckwandler 10 beaufschlagt. Für dieses Druckuntersetzungsverhältnis wird zweckmäßigerweise ein Wert von beispielsweise 0,8 bis 0,95 gewählt, in jedem Fall aber ein Wert, der bei Umschaltung auf eine niedrigere Druckstufe eine sichere Öffnung des Reduzierventils 23 gewährleistet. In der Praxis hat sich eine Reduzierung ca. 10 %, d. h. ein Untersetzungsverhältnis von ca. 0,9, als ausreichend und brauchbar erwiesen. Der zweite Komparator 21 hat also einerseits die Aufgabe, bei Umschaltung des Setzwerts der Druckvorwahl das Reduzierventil 23 so lange zu öffnen, wie der Druck in der Pelotte den neu eingestellten Sollwert um den Differenzwert zwischen diesem Sollwert und dem reduzierten Drucksignal überschreitet. Selbstverständlich wird die vollständige Entleerung der Pelotte bei Abschaltung des Geräts ebenfalls über das Reduzierventil 23 erfolgen. Der zweite Komparator 21 stellt aber andererseits auch eine Überwachung für den ersten Komparator 12 dar. Denn übersteigt der Druck in der Pelotte 7 den gewählten Setzwert des Druck aus irgendeinem Grund um einen bestimmten, durch das Reduzierverhältnis festgelegten Betrag, so liefert der zweite Komparator 21 über die Impulselektronik 22 das Signal zum Öffnen des Reduzierventils 23.

Zur zusätzlichen Sicherheit für das Auge gegen Überdruck im Falle eines vollständigen Defekts der Elektronik, d.h. bei eventuellem Dauerlauf der Pumpe 14 oder bei Versagen des elektromagnetischen Reduzierventils 23 ist im Drucksystem außerdem noch ein (nicht gezeigtes) mechanisches Ventil vorgesehen, das bei einem Druck ab 55 mmHg automatisch öffnet, so daß eine Schädigung des Auges in jedem Fall ausgeschlossen ist.

Durch die Erfindung wird die Okulopression eine größere Akzeptanz in der Ophthalochirurgie erhalten, da die bisherigen Nachteile, wie schwierige manuelle Druckregelung, Überdruckgefahr, Bedarf von erfahrenem und geschultem Personal zur Überwachung, beseitigt sind. Es wird eine höhere Sicherheit, Entlastung des Personals und vor allem eine bisher nicht erzielte Wirksamkeit mit einer vollkommen automatischen Druckkonstanthaltung für die Okulopression erreicht.

## Patentansprüche

1. Okulopressionsgerät mit einer an eine Pumpe (14) angeschlossenen und auf dem Auge eines Probanden zu befestigenden Pelotte (7), gekennzeichnet durch eine elektronische Steuer- und Überwachungseinrichtung mit
— einer Schaltereinrichtung (3, 20) zur Vorgabe eines Sollwerts, der einem gewünschten Druckwert in der Pelotte (7) entspricht,
— einem Druckwandler (10), der den von der Pumpe (14) in der Pelotte (7) erzeugten Druck in ein entsprechendes elektrisches Drucksignal umsetzt,
— einer Vergleichseinrichtung mit zwei Komparatoren (12, 21), von denen der eine (12) eingangsseitig einerseits mit dem vorgegebenen Sollwert von der Schaltereinrichtung (20) und andererseits mit dem Drucksignal beaufschlagt ist und ausgangsseitig ein Treibersignal an die Pumpe (14) zur Druckerhöhung in der Pelotte (7) liefert, solange das Drucksignal unter dem Sollwert liegt oder bei Druckabfall in der Pelotte (7) um einen geringen Betrag, und von denen der andere (21) eingangsseitig einerseits mit dem Sollwert und andererseits mit einem um ein festes Verhältnis reduzierten Anteil des Drucksignals beaufschlagt ist und ausgangsseitig ein Steuersignal an ein im Verbindungskanal zwischen der Pumpe (14) und der Pelotte (7) liegendes Reduzierventil (23) liefert, sobald der Druck in der Pelotte (7) den Sollwert um den Differenzwert zwischen dem Sollwert und dem reduzierten Drucksignal überschreitet, und durch
— eine elektrische Anzeigeeinrichtung (2), welche das Drucksignal anzeigt.

2. Okulopressionsgerät nach Anspruch 1, dadurch gekennzeichnet, daß das feste Reduktionsverhältnis für das Druckwandlersignal im Bereich von 0,8 bis 0,95 und vorzugsweise zu etwa 0,9 gewählt ist.

3. Okulopressionsgerät nach Anspruch 1, dadurch gekennzeichnet, daß der feste Reduktionsbetrag für das Druckwandlersignal 400 bis 533 Pa (3 bis 4 mmHg) entspricht.

4. Okulopressionsgerät nach Anspruch 1, dadurch gekennzeichnet, daß im Drucksystem (14, 9) des Geräts zur Bekämpfung von Druckschwankungen nach oben und unten ein Zusatzvolumen eingebaut ist, das mindestens dem Volumen der Pelotte (7) entspricht.

5. Okulopressionsgerät nach Anspruch 1, dadurch gekennzeichnet, daß die Schaltereinrichtung (3, 20) zur Nullpunktkontrolle des Druckwandlers (10) nach dem Einschalten erst auf den Druckwert Null geschaltet werden muß.

6. Okulopressionsgerät nach einem der vorstehenden Ansprüche, dadurch gekennzeichnet, daß im Drucksystem zur Sicherheit des Probanden ein mechanisches Überdruck-Sicherheitsventil eingebaut ist.

## Claims

1. Ocular pressure apparatus with a spherical pressure pad (7) attached to a pump (14) and to be mounted on the eye of a person being observed, characterized by an electronic control and monitoring device having
— a switching device (3, 20) for the stipulation of a desired value which corresponds to a desired pressure value in the spherical pressure pad (7),
— a pressure transducer (10) which converts the pressure, produced by the pump (14) in the spherical pressure pad (7), into a corresponding electrical pressure signal,
— a comparison device with two comparators (12, 21), of which the one (12) on the input side is loaded on the one hand with the predetermined desired value by the switching device (20) and on the other hand is loaded with the pressure signal and on the output side supplies a driving signal to the pump (14) to increase pressure in the spherical pressure pad (7) as long as the pressure signal lies below the desired value or with a drop in pressure in the spherical pressure pad (7) by a small amount, and of which the other comparator (21) on the input side on the one hand is loaded with the desired value and on the other hand is loaded with a portion of the pressure signal reduced by a fixed ratio, and on the output side supplies a control signal to a reducing valve (23) lying in the connecting channel between the pump (14) and the spherical pressure pad (7) as soon as the pressure in the spherical pressure pad (7) exceeds the desired value by the difference value between the desired value and the reduced pressure signal, and by
— an electrical indicating device (2) which indicates the pressure signal.

2. Ocular pressure apparatus according to claim 1, characterized in that the fixed reduction ratio for the pressure transducer signal is chosen in the region of 0.8 to 0–95 and preferably to about 0.9.

3. Ocular pressure apparatus according to claim 1, characterized in that the fixed reduction amount for the pressure transducer signal corresponds to 400 to 533 Pa (3 to 4 mmHg).

4. Ocular pressure apparatus according to claim 1, characterized in that in the pressure system (14, 9) of the apparatus for the damping of fluctuations of pressure upwards and downwards an additional volume is incorporated, which corresponds at least to the volume of the spherical pressure pad (7).

5. Ocular pressure apparatus according to claim 1, characterized in that after the switching on, the switching device (3, 20) for zero point control of the pressure transducer (10) must at first be connected to the pressure value zero.

6. Ocular pressure apparatus according to one of the preceding claims, characterized in that a mechanical excess pressure safety valve is incorporated into the pressure system for the safety of the person being observed.

## Revendications

1. Instrument d'application d'une pression intra-oculaire avec une pelote (7) raccordée à une pompe (14) et devant être fixée sur l'oeil d'un patient, caractérisé en ce qu'il comprend un dispositif de commande et de surveillance électronique avec
   – un dispositif de commutation (3, 20) pour la prédétermination d'une valeur de consigne qui correspond à une valeur de pression désirée dans la pelote (7),
   – un convertisseur de pression (10) qui transforme la pression créée par la pompe (14) dans la pelote (7) en un signal de pression électrique correspondant,
   – un dispositif de comparaison avec deux comparateurs (12, 21) dont l'un (12) reçoit du côté entrée la valeur de consigne prédéterminée par le dispositif de commutation (20), d'une part, et le signal de pression, d'autre part, et délivre du côté sortie un signal d'attaque pour la pompe (14) en vue de l'augmentation de la pression reste inférieur à la valeur de consigne ou en cas d'une faible chute de pression dans la pelote (7), et dont l'autre (21) reçoit du côté entrée la valeur de consigne, d'une part, et une partie du signal de pression diminuée d'un rapport fixe, d'autre part, et délivre du côté sortie un signal de commande pour un détendeur (23) placé dans le canal de connexion entre la pompe (14) et la pelote (7) dès que la pression à l'intérieur de la pelote (7) dépasse la valeur de consigne de la valeur différentielle entre la valeur de consigne et le signal de pression diminué, et
   – un indicateur électrique (2) qui affiche le signal de pression.

2. Instrument d'application d'une pression intra-oculaire selon la revendication 1, caractérisé en ce que le rapport de réduction fixe pour le signal du convertisseur de pression est choisi dans la plage de 0,8 à 0,95 et qu'il est de préférence de 0,9 environ.

3. Instrument d'application d'une pression intra-oculaire selon la revendication 1, caractérisé en ce que le rapport de réduction fixe pour le signal du convertisseur de pression correspond à 400 à 533 Pa (3 à 4 mmHg).

4. Instrument d'application d'une pression intra-oculaire selon la revendication 1, caractérisé en ce que pour s'opposer à des variations de pression vers le haut et vers le bas, le système de pression (14, 9) comprend un volume supplémentaire qui correspond au moins au volume de la pelote (7).

5. Instrument d'application d'une pression intra-oculaire selon la revendication 1, caractérisé en ce que, pour le contrôle du point zéro du convertisseur de pression (10), le dispositif de commutation (3, 20) doit tout d'abord, après la mise en circuit, être commuté sur la valeur de pression zéro.

6. Instrument d'application d'une pression intra-oculaire selon l'une des revendications précédentes, caractérisé en ce qu'une soupape de surpression mécanique est montée dans le système de pression pour la sécurité.

**Fig. 1**

EP 0 266 451 B1

Fig 2

Druck-wandler `10` — Verstärker `11`

Erster Komparator `12` — Treiber `13` — Pumpe `14`

Anzeige `2`

`9` ↓ an Pelotte

Druck-vorwahl (Schalter) `20`

Zweiter Komparator `21` — Impuls-Elektronik `22` — Ventil `23`

↓ an Umgebung

Batterie `25` — Komparator 3 `26` — LED Batterie-Kontrolle `5`

Regler `27` → Betriebsspannung

EP 0 266 451 B1